(19) Europäisches Patentamt
European Patent Office
Office européen des brevets

(11) **EP 4 722 504 A1**

(12) **EUROPEAN PATENT APPLICATION**
published in accordance with Art. 153(4) EPC

(43) Date of publication:
08.04.2026 Bulletin 2026/15

(21) Application number: 24815512.9

(22) Date of filing: 29.05.2024

(51) International Patent Classification (IPC):
*F01M 11/10* (2006.01)     *F01M 11/12* (2006.01)
*F16N 29/00* (2006.01)     *G01N 33/30* (2006.01)

(52) Cooperative Patent Classification (CPC):
F01M 11/10; F01M 11/12; F16N 29/00; G01N 33/30

(86) International application number:
PCT/JP2024/019673

(87) International publication number:
WO 2024/248031 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.05.2023 JP 2023088047

(71) Applicant: NSK LTD.
Tokyo 141-8560 (JP)

(72) Inventors:
• NAKAGAWA, Kazunori
Fujisawa-shi, Kanagawa 251-8501 (JP)
• MAEDA, Masayuki
Fujisawa-shi, Kanagawa 251-8501 (JP)
• MARUYAMA, Taisuke
Fujisawa-shi, Kanagawa 251-8501 (JP)

(74) Representative: Grünecker Patent- und
Rechtsanwälte
PartG mbB
Leopoldstraße 4
80802 München (DE)

(54) **METHOD FOR CALCULATING EVAPORATION AMOUNT OF LUBRICANT, CALCULATION DEVICE, AND PROGRAM**

(57) This method for calculating the evaporation amount of a lubricant within a mechanical device to the exterior includes: a calculation step in which the evaporation amount of the lubricant is calculated using a calculation formula defined so as to include a container shape parameter defined by modeling the configuration within the machine device as a container in which the lubricant is accommodated, a physical property parameter of the lubricant, an environmental parameter from the area around the lubricant, and a parameter regarding time; and an output step in which the evaporation amount calculated in the calculation step is output.

*FIG. 2*

(a)          (b)

**Description**

TECHNICAL FIELD

The present invention relates to a method, a device, and a program for calculating an evaporation amount of a lubricant.

BACKGROUND ART

In the related art, in a mechanical device such as a bearing device or a sliding device, a lubricant is used for the purpose of reducing friction in an operation of the mechanical device. An amount of the lubricant in the mechanical device may vary depending on a device configuration, elapse of time, and the like. Since a change in the amount of the lubricant affects the operation of the mechanical device, it is important to manage the amount of the lubricant in the mechanical device in order to appropriately operate the mechanical device.

For example, Patent Literature 1 discloses a configuration of an open rolling bearing that prevents a lubricating oil from entering a bearing space due to oil plating. With such a configuration, in Patent Literature 1, a variation of the amount of the lubricant in the bearing space is prevented, and a torque variation is prevented while maintaining a lubrication state of the bearing space.

CITATION LIST

PATENT LITERATURE

Patent Literature 1: JP2007-107588A

SUMMARY OF INVENTION

TECHNICAL PROBLEM

It is assumed that the lubricant in the mechanical device evaporates with elapse of time and flows out of the mechanical device. Therefore, as a result of the lubricant flowing out of the mechanical device due to evaporation, the amount of the lubricant in the mechanical device decreases, which may affect the operation of the mechanical device. In Patent Literature 1, entry of the lubricating oil into the bearing space is considered, but the variation in the amount related to the evaporation of the lubricant is not considered.

In view of the above problems, an object of the present invention is to provide a method for calculating an evaporation amount of a lubricant in a mechanical device.

SOLUTION TO PROBLEM

In order to solve the above problem, the present invention has the following configuration. That is, a method for calculating an evaporation amount of a lubricant in a mechanical device to outside, the method including:

a calculation step of calculating the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and
an output step of outputting the evaporation amount calculated in the calculation step.

In addition, another aspect of the present invention has the following configuration. That is, a device for calculating an evaporation amount of a lubricant in a mechanical device to outside, the device including:

a calculation unit configured to calculate the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and
an output unit configured to output the evaporation amount calculated by the calculation unit.

In addition, another aspect of the present invention has the following configuration. That is, a program that causes a computer to execute:

a calculation step of calculating an evaporation amount of a lubricant to outside using a calculation formula defined by including a container shape parameter defined by modeling a configuration in a mechanical device as a container in which the lubricant is accommodated, a physical property parameter of the lubricant, an environmental parameter around the lubricant, and a parameter of time; and
an output step of outputting the evaporation amount calculated in the calculation step.

ADVANTAGEOUS EFFECTS OF INVENTION

According to the present invention, it is possible to calculate an evaporation amount of a lubricant in a mechanical device.

BRIEF DESCRIPTION OF DRAWINGS

FIG. 1 is a conceptual diagram illustrating a change in an amount of a lubricant according to an embodiment of the present invention.
FIG. 2 is a diagram illustrating an example of a measurement result of the lubricant according to the embodiment of the present invention.
FIG. 3 is a diagram illustrating an example of the measurement result of the lubricant according to the embodiment of the present invention.
FIG. 4 is a diagram illustrating a case where a calculation method according to the embodiment of the present invention is applied to a rolling bearing.
FIG. 5 is a model diagram illustrating a case where the calculation method according to the embodiment of the present invention is applied to a rolling bearing.
FIG. 6 is a model diagram illustrating a case where the calculation method according to the embodiment of the present invention is applied to a rolling bearing.
FIG. 7 is a graph illustrating an example of a measurement result of a variation amount of the rolling bearing by the calculation method according to the embodiment of the present invention.
FIG. 8 is a block diagram illustrating a configuration example of an information processing device capable of executing the calculation method according to the embodiment of the present invention.

DESCRIPTION OF EMBODIMENTS

Hereinafter, embodiments of the present invention will be described with reference to the drawings. The embodiments described below are embodiments for explaining the present invention, and are not intended to be interpreted to limit the present invention, and all the configurations described in the embodiments are not necessarily essential configurations for solving the problem of the present invention. In the drawings, the same components are denoted by the same reference numerals, thereby showing a correspondence relation therebetween.

<First Embodiment>

Hereinafter, a first embodiment of the present invention will be described. In the present embodiment, a rolling bearing will be described as an example of a mechanical device that contains a lubricant therein, the lubricant being used for operation. However, the present invention is not limited thereto, and as will be described later, is applicable to any device including a path through which the lubricant evaporates and flows to outside.

In the case of a rolling bearing, an open configuration in which a path to an external space is formed around the lubricant is assumed. Examples of a type of the rolling bearing include a deep groove ball bearing, an angular contact ball bearing, a tapered roller bearing, a cylindrical roller bearing, and a self-aligning roller bearing. Examples of other mechanical devices using a lubricant include a gear device, a reduction gear, a rotating device such as a wind turbine, a sliding device that performs a sliding operation, and a motor, and the present invention is also applicable to these devices.

[Change in Amount of Lubricant]

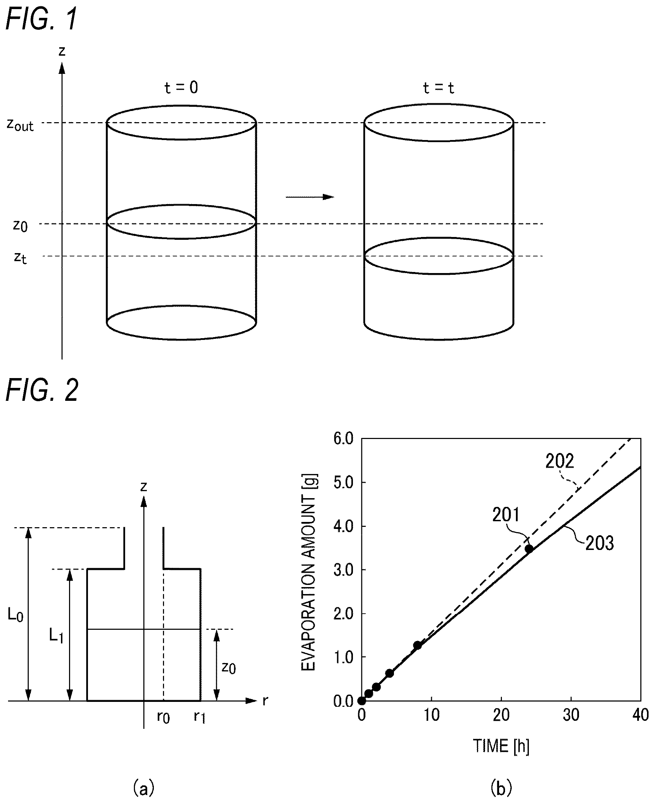

FIG. 1 is a conceptual diagram illustrating a change in an amount of the lubricant in the mechanical device according to the present embodiment. The lubricant according to the present embodiment has a property of evaporating with elapse of time. In addition, it is assumed that the lubricant is accommodated in a container having a predetermined shape, and in the example of FIG. 1, a cylindrical container will be described as an example.

In FIG. 1, a vertical axis represents a height of a liquid level of the lubricant with reference to a bottom surface of the container. A liquid level height of the lubricant at initial time (t = 0) is indicated by $z_0$, and the liquid level height of the lubricant at a time point when time t elapses is indicated by $z_t$. A height of an opening of the container in which the lubricant is accommodated is indicated by $z_{out}$. That is, the lubricant evaporates with elapse of time and flows to outside through the opening of the container. As a result, when the time t elapses, the liquid level height changes from $z_0$ to $z_t$.

A relation between the liquid level height and the time as illustrated in FIG. 1 can be expressed by following Formula (1).

[Math. 1]

$$-\frac{MD}{\rho RT}P_{vap}t = \int_{z_0}^{z_t}\left\{\left(\int_z^{z_{out}}\frac{dz}{S(z)}\right)S(z)\right\}dz \quad \cdots \quad (1)$$

M: molecular weight [g/mol] of lubricant
D: diffusion coefficient [m$^2$/s]
$\rho$: density of lubricant [g/m$^3$]
R: gas constant [J·K$^{-1}$mol$^{-1}$]
T: temperature [K]
$P_{vap}$: saturated vapor pressure [Pa] at temperature T
t: time [s]
$z_t$: liquid level height [m] at time t
$z_0$: liquid level height [m] at initial time
z: liquid level height [m] at any time ($z_t < z < z_0$)
$z_{out}$: height [m] of opening of container
S(z): cross-sectional area [m$^2$] of container at z

In addition, a relation between an evaporation amount of the lubricant and the time can be expressed by following Formula (2).

[Math. 2]

$$w = -\rho\int_{z_0}^{z_t}S(z)\,dz \quad \cdots \quad (2)$$

w: evaporation amount [g]
$\rho$: density of lubricant [g/m$^3$]
$z_t$: liquid level height [m] at time t
$z_0$: liquid level height [m] at initial time
z: liquid level height [m] at any time ($z_t < z < z_0$)
S(z): cross-sectional area [m$^2$] of container at z

A change in the liquid level height and the evaporation amount are predicted by Formulas (1) and (2). In the present embodiment, outflow of the lubricant to the outside of the container by vaporization or the like is referred to as "evaporation". On the other hand, the lubricant is treated as not being evaporated when it remains in the container in a case of being in a vaporized state.

Further, when the evaporation amount of the lubricant is small, that is, when lowering of the liquid level of the lubricant can be ignored, that is, when the condition represented by following Formula (3) is satisfied, Formula (1) and Formula (2) can be integrated into following Formula (4).

[Math. 3]

$$\int_z^{z_{out}}\frac{dz}{S(z)} = \int_{z_0}^{z_{out}}\frac{dz}{S(z)} \quad \cdots \quad (3)$$

[Math. 4]

$$w = \left( \int_{z_0}^{z_{out}} \frac{dz}{S(z)} \right)^{-1} \cdot \frac{MDP_{vap}}{RT} \cdot t \quad \cdots \quad (4)$$

w: evaporation amount [g]
$z_{out}$: height [m] of opening of container
$z_0$: liquid level height [m] at initial time
M: molecular weight [g/mol] of lubricant
D: diffusion coefficient [$m^2$/s]
R: gas constant [$J \cdot K^{-1} mol^{-1}$]
T: temperature [K]
$P_{vap}$: saturated vapor pressure [Pa] at temperature T
t: time [s]
z: liquid level height [m] at any time ($z_t < z < z_0$)
S(z): cross-sectional area [$m^2$] of container at z

In Formula (4), attention is paid to three parts. First, in Formula (4), the following part is a parameter related to a shape of the container in which the lubricant is accommodated (hereinafter, referred to as "container shape parameter").

[Math. 5]

$$\int_{z_0}^{z_{out}} \frac{dz}{S(z)}$$

In Formula (4), an "$MDP_{vap}$" part is a parameter related to physical properties of a vapor of the lubricant (hereinafter, referred to as "physical property parameter"). Further, in Formula (4), a "RT" part is a parameter related to an environment around the lubricant (hereinafter, referred to as "environmental parameter").

That is, the evaporation amount of the lubricant may be calculated by specifying the physical properties of the lubricant and dimensions of the container in which the lubricant is accommodated, which are defined in advance in Formula (4). In the present embodiment, Formula (1) and Formula (2) are referred to as "non-approximate formula", Formula (4) is referred to as "approximate formula", and measurement results will be described. The non-approximate formula and the approximate formula also include the parameter of time, more specifically, a parameter indicating elapse of time from a reference.

In the case of the non-approximate formula, the container shape parameter is shown on a right side of Formula (1), and the physical property parameter and the environmental parameter are shown on a left side.

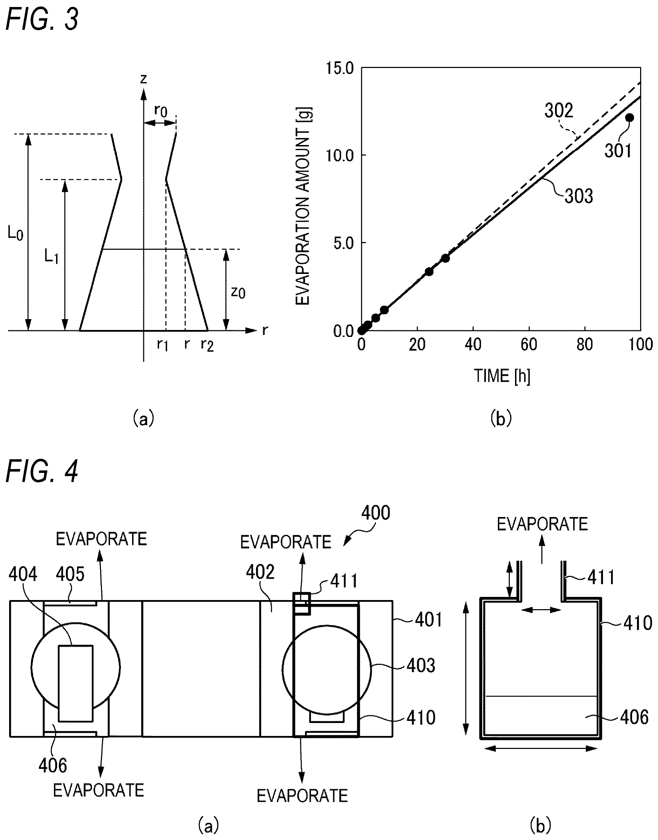

FIGS. 2 and 3 are diagrams illustrating comparison between a calculated value by the method according to the present embodiment and an actually measured value by taking two container shapes as an example. Here, hexane is used as the lubricant, and a test is performed at a test temperature of 25°C.

(a) of FIG. 2 illustrates an example of a shape of a container in which the lubricant is accommodated. In the example of the container illustrated in (a) of FIG. 2, a main body has a cylindrical shape, and an opening has a cylindrical shape with a smaller diameter than the main body. A radius of the main body of the container is indicated by $r_1$, and a radius of the opening is indicated by $r_0$ ($< r_1$). A height of the main body is indicated by $L_1$, and a height to an uppermost portion of the container is indicated by $L_0$ ($> L_1$). Therefore, a length of the opening in a height direction is $L_0$-$L_1$. Further, the lubricant is accommodated in the container, and an initial liquid level height of the lubricant is indicated by $z_0$ ($< L_1$). To simplify the description, a thickness of the container is ignored here.

(b) of FIG. 2 is a graph illustrating an example of the calculated value by the method according to the present embodiment and the actually measured value based on the configuration in (a) of FIG. 2. In (b) of FIG. 2, a vertical axis represents the evaporation amount [g], and a horizontal axis represents time [h]. A plot 201 indicates the actually measured value. A broken line 202 indicates a calculated value by Formula (3). A solid line 203 indicates calculated values by Formulas (1) and (2).

As illustrated in FIG. 2, it is possible to calculate, by the method according to the present embodiment, the evaporation amount with elapse of time with high accuracy with respect to the actually measured value.

(a) of FIG. 3 illustrates another example of the shape of the container in which the lubricant is accommodated. In the example of the container illustrated in (a) of FIG. 3, a main body has a tapered shape tapered toward an opening like a conical flask, and the opening has a tapered shape tapered toward an uppermost portion contrary to the main body. A radius of a bottom surface of the container is indicated by $r_2$, a radius of a connection portion between the main body and the opening is indicated by $r_1$ ($<r_2$), and a radius of the opening of the container is indicated by $r_0$ ($> r_1$). A height of the main body is indicated by $L_1$, and a height to the uppermost portion of the container is indicated by $L_0$ ($> L_1$). Therefore, a length of the opening in a height direction is $L_0$-$L_1$. Further, the lubricant is accommodated in the container, and an initial liquid level height of the lubricant is indicated by $z_0$ ($< L_1$). To simplify the description, a thickness of the container is ignored here.

(b) of FIG. 3 is a graph illustrating an example of the calculated value by the method according to the present embodiment and the actually measured value based on the configuration in (a) of FIG. 3. In (b) of FIG. 3, a vertical axis represents the evaporation amount [g], and a horizontal axis represents time [h]. A plot 301 indicates the actually measured value. A broken line 302 indicates a calculated value by Formula (3). A solid line 303 indicates calculated values by Formulas (1) and (2).

As in the example illustrated in FIG. 2, even when the shape of the container changes, the method according to the present embodiment is applicable, and it is possible to calculate the evaporation amount with elapse of time with high accuracy with respect to the actually measured value.

[Example of Application to Mechanical Device]

An example in which the above-described calculation method is applied to a mechanical device will be described. Here, a rolling bearing will be described as an example of the mechanical device as a predict target.

FIG. 4 is a conceptual diagram of the rolling bearing that calculates an evaporation amount of a lubricant according to the present embodiment. As illustrated in (a) of FIG. 4, a rolling bearing 400 includes an outer ring 401, an inner ring 402, rolling elements 403, a cage 404, a seal 405, and a lubricant 406. The lubricant 406 is filled to reduce friction between the outer ring 401 and the rolling element 403 and between the inner ring 402 and the rolling element 403 and performs lubrication. The seal 405 prevents impurities such as dust from entering the rolling bearing 400. The rolling bearing 400 is provided with a gap between the seal 405 and the inner ring 402 for a rotational operation thereof, and the lubricant 406 may flow out, that is, evaporate from the gap.

In the present embodiment, an example in which the inner ring 402 is a rotating ring and the outer ring 401 is a fixed ring is illustrated, but the configuration may be reversed. In addition, the seal 405 may be installed on an inner ring 402 side, and a gap may be provided between the outer ring 401 and the seal 405.

(b) of FIG. 4 illustrates a model of a container that accommodates the lubricant 406 in the rolling bearing 400. In the present embodiment, as indicated by a frame line 410, a part surrounded by the outer ring 401, the inner ring 402, and the seal 405 in the rolling bearing 400 is assumed as a main body of the container. Further, as indicated by a frame line 411, a gap formed between the inner ring 402 and the seal 405 is assumed as an opening of the container. In FIG. 4, although a part is simplified, an area of the gap formed between the inner ring 402 and the seal 405 corresponds to an area of an uppermost surface of the opening of the container.

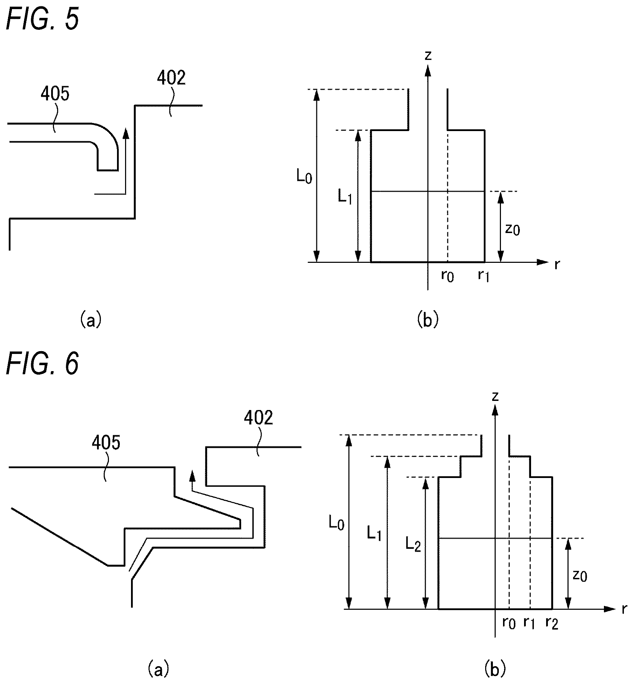

FIG. 5 is an enlarged view of a part of the inner ring 402 and the seal 405 in the rolling bearing 400. Here, an example of a single-row deep groove ball bearing (number: 608) is illustrated. As indicated by an arrow in (a) of FIG. 5, a flow path (opening) for the lubricant is formed in the gap between the inner ring 402 and the seal 405, and the lubricant flows to the outside through the flow path. (b) of FIG. 5 illustrates a model of the container shape based on the configurations in (a) of FIG. 4 and (a) of FIG. 5. This example is the same as that illustrated in (a) of FIG. 2.

FIG. 6 is another enlarged view of a part of the inner ring 402 and the seal 405 in the rolling bearing 400. Here, an example of a single-row deep groove ball bearing (number: 6203) is illustrated. A shape of the flow path (opening) formed between the inner ring 402 and the seal 405 is different from the example of FIG. 5. (b) of FIG. 6 illustrates a model of the container shape based on the configurations in (a) of FIG. 4 and (a) of FIG. 6. The shape of the opening is modeled to be different from that in FIG. 5, and the radii $r_0$, $r_1$, and $r_2$ and the lengths $L_0$, $L_1$, and $L_2$ are set corresponding to a change in the diameter and the length of the flow path.

The modeling, that is, the container shape parameter may be defined according to the configuration of the mechanical device as a predict target.

[Example of Test Result]

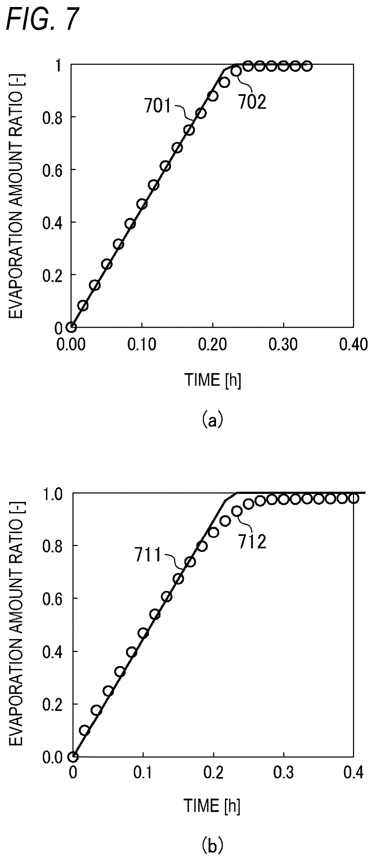

FIG. 7 is a graph illustrating a calculation result by the method according to the present embodiment and the actually measured value based on the modeling of the rolling bearing illustrated in FIGS. 5 and 6. In FIG. 7, a vertical axis represents an evaporation amount ratio (ratio of evaporation amount to initial total amount of lubricant), and a horizontal axis represents the time [h]. In this example, a calculation result of the non-approximation formulas by Formulas (1) and (2) is illustrated.

Test conditions are as follows. In this example, an organic solvent is used as the lubricant in order to ignore a change in the physical property parameter due to oxidation. The rolling bearing used in the test is in a stationary state.

(Physical Properties of Organic Solvent)

Organic solvent: hexane
Molecular weight: 86.18 [g/mol]
Saturated vapor pressure (21°C): $1.7 \times 10^4$ [Pa]
Saturated vapor pressure (25°C): $2.0 \times 10^4$ [Pa]
Collision diameter: 0.53 [nm]

(Test Environment and Physical Properties)

Atmosphere: air
Molecular weight: 28.96 [g/mol]
Collision diameter: 0.3617 [nm]
Atmospheric pressure: $1.01325 \times 10^5$ [Pa]
Temperature: room temperature (°C)

(a) of FIG. 7 corresponds to an example of the single-row deep groove ball bearing (number: 608) illustrated in FIG. 5. Here, a result at the room temperature of 21°C as the temperature is illustrated. A solid line 701 indicates the calculation result by the method according to the present embodiment. A plot 702 indicates the actually measured value of the evaporation amount. (b) of FIG. 7 corresponds to an example of the single-row deep groove ball bearing (number: 6203) illustrated in FIG. 6. Here, a result at the room temperature of 25°C as the temperature is illustrated. A solid line 711 indicates the calculation result by the method according to the present embodiment. A plot 712 indicates the actually measured value of the evaporation amount.

As illustrated in FIG. 7, the evaporation amount of the lubricant may be predicted with high accuracy.

The method according to the present embodiment may measure the evaporation amount of the lubricant contained in an already-produced mechanical device as a target. As another application, the method according to the present embodiment may also be used at the time of predicting the evaporation amount in order to satisfy a target condition of the evaporation amount in the design of the mechanical device. Therefore, in the method according to the present embodiment, a timing of use is also not particularly limited in addition to a type of the device as a target.

Whether to use Formulas (1) and (2), which are non-approximate formulas, or Formula (4), which is an approximate formula, may be switched based on conditions at the time of performing calculation. For example, when higher accuracy is required, a non-approximate formula may be used.

According to Formula (4), the container shape parameter uses a reciprocal of an integral of a cross-sectional area of the path. Therefore, as the path becomes narrower, the parameter becomes larger, and the influence of the narrow path becomes larger. For example, in the design of the mechanical device, when it is intended to reduce the evaporation amount of the lubricant, the gap between the seal and the inner ring may be designed such that the flow path of the lubricant is narrowed, that is, the container shape parameter is increased.

[Device Configuration]

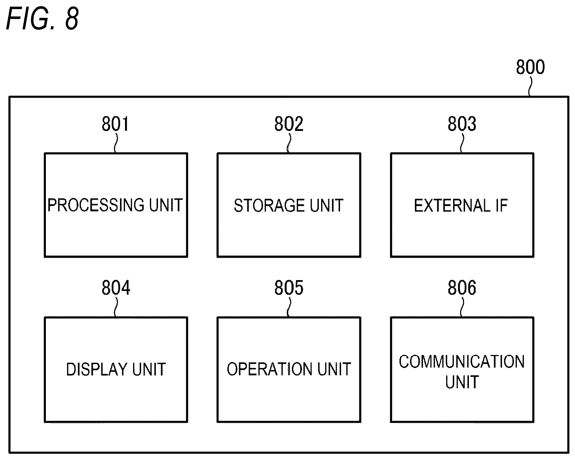

FIG. 8 is a diagram illustrating a configuration example of an information processing device capable of measuring or predicting evaporation of a lubricant of a mechanical device using the method according to the present embodiment. An information processing device 800 includes a processing unit 801, a storage unit 802, an external IF (interface) 803, a display unit 804, an operation unit 805, and a communication unit 806. The information processing device 800 may be implemented as, for example, a general-purpose information processing device such as a personal computer (PC) or may be implemented as a dedicated device.

The processing unit 801 may include a central processing unit (CPU), a micro processing unit (MPU), a digital single processor (DSP), or a dedicated circuit. The storage unit 802 includes volatile and nonvolatile storage media such as a hard disk drive (HDD), a read only memory (ROM), and a random access memory (RAM), and may input and output various kinds of information in response to an instruction from the processing unit 801. The processing unit 801 may implement the above-described calculation method by reading and executing a program, an application, and various data related to the calculation method according to the present embodiment from the storage unit 802.

The storage unit 802 holds, for example, the calculation formulas shown in above Formulas (1), (2), and (4) and various parameters of the mechanical device as a measurement target. More specifically, the container shape parameter, the physical property parameter, the environmental parameter, and the like are held.

The external IF 803 is an interface for connecting to an external device. For example, when the method according to the present embodiment is used at the time of measurement, the external IF 803 may be connected to a sensor or the like for measuring information such as a temperature or an atmospheric pressure in a surrounding environment of the measurement target. The display unit 804 is a part for outputting a result obtained by the method according to the present embodiment, and outputs the result to a user according to an instruction from the processing unit 801. The operation unit 805 is a part for receiving input of parameters and input of various instructions from the user. The communication unit 806 is a network interface for communicating with an external device, and may output the result obtained by the method according to the present embodiment to the outside, for example.

For example, in the present embodiment, it is possible to use a non-approximate formula and an approximate formula as described above. Therefore, the user may input, via the operation unit 805, a switching instruction to enable calculation using any of these formulas. In addition, a plurality of pieces of dimensional information modeled around the lubricant in the mechanical device as a measurement target may be received via the operation unit 805 and the communication unit 806.

As described above, according to the configuration of the present embodiment, it is possible to provide a method for calculating an evaporation amount of a lubricant in a mechanical device.

<Other Embodiments>

In the present invention, a program or an application for implementing functions of the one or more embodiments described above may be supplied to a system or a device using a network or a storage medium, and a process in which one or more processors in a computer of the system or the device read and execute the program may be implemented.

In addition, it may be implemented by a circuit (for example, application specific integrated circuit (ASIC) or field programmable gate array (FPGA)) that implements one or more functions.

As described above, the present invention is not limited to the above-described embodiments, and combinations of the respective configurations of the embodiments and changes and modifications made by those skilled in the art based on the descriptions in the description and the well-known technique are intended by the present invention and are thus also included within the scope of the present invention to be protected.

As described above, the following matters are disclosed in the present description.

(1) A method for calculating an evaporation amount of a lubricant in a mechanical device to outside, the method including:

a calculation step of calculating the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and
an output step of outputting the evaporation amount calculated in the calculation step.
According to this configuration, it is possible to provide a method for calculating an evaporation amount of a lubricant in a mechanical device.

(2) The calculation method according to (1), in which

the calculation formula is defined by the following formula.

[Math. 6]

$$-\frac{MD}{\rho RT}P_{vap}t = \int_{z_0}^{z_t}\left\{\left(\int_z^{z_{out}}\frac{dz}{S(z)}\right)S(z)\right\}dz$$

$$w = -\rho\int_{z_0}^{z_t}S(z)\,dz$$

w: evaporation mass [g]
M: molecular weight [g/mol] of lubricant

D: diffusion coefficient [m$^2$/s]

$\rho$: density of lubricant [g/m$^3$]

R: gas constant [J·K$^{-1}$mol$^{-1}$]

T: temperature [K]

$P_{vap}$: saturated vapor pressure [Pa] at temperature T

t: time [s]

$z_t$: liquid level height [m] at time t

$z_0$: liquid level height [m] at initial time

z: liquid level height [m] at any time ($z_t < z < z_0$)

$z_{out}$: height [m] of opening of container

S(z): cross-sectional area [m$^2$] of container at z

According to this configuration, it is possible to calculate an evaporation amount of a lubricant in a mechanical device with higher accuracy.

(3) The calculation method according to (1), in which

the calculation formula is defined by the following formula.

[Math. 7]

$$w = \left( \int_{z_0}^{z_{out}} \frac{dz}{S(z)} \right)^{-1} \cdot \frac{MDP_{vap}}{RT} \cdot t$$

w: evaporation mass [g]

$z_{out}$: height [m] of opening of container

$z_0$: liquid level height [m] at initial time

M: molecular weight [g/mol] of lubricant

D: diffusion coefficient [m$^2$/s]

R: gas constant [J·K$^{-1}$mol$^{-1}$]

T: temperature [K]

$P_{vap}$: saturated vapor pressure [Pa] at temperature T

t: time [s]

z: liquid level height [m] at any time

S(z): cross-sectional area [m$^2$] of container at z

According to this configuration, it is possible to calculate an evaporation amount of a lubricant in a mechanical device by a simpler calculation formula.

(4) The calculation method according to any of (1) to (3), in which

the evaporation amount of the lubricant is an amount of the lubricant flowing out of the container.

According to this configuration, it is possible to calculate an evaporation amount in consideration of a lubricant that is evaporated and accumulated in a device.

(5) The calculation method according to any of (1) to (4), in which

the mechanical device is a rolling bearing, a rotating device, or a sliding device.

According to this configuration, it is possible to calculate an evaporation amount for various mechanical devices using a lubricant.

(6) A method for designing a mechanical device using the calculation method according to any of (1) to (5), the method including:

a receiving step of receiving designation of a container shape parameter defined by modeling a configuration in the mechanical device as a container in which the lubricant is accommodated.

According to this configuration, it is possible to perform design in consideration of an evaporation amount of a lubricant in a design stage of a mechanical device.

(7) A method for measuring an evaporation amount of a lubricant in a mechanical device using the calculation method according to any of (1) to (5), the method including:

an acquisition step of acquiring the environmental parameter around the lubricant of the mechanical device, the physical property parameter of the lubricant, and the parameter of time.

According to this configuration, an evaporation amount of a lubricant inside an existing mechanical device may be measured without actual measurement.

(8) A device for calculating an evaporation amount of a lubricant in a mechanical device to outside, the device including:

a calculation unit configured to calculate the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and

an output unit configured to output the evaporation amount calculated by the calculation unit.

According to this configuration, it is possible to provide a device for calculating an evaporation amount of a lubricant in a mechanical device.

(9) A program that causes a computer to execute:

a calculation step of calculating an evaporation amount of a lubricant to outside using a calculation formula defined by including a container shape parameter defined by modeling a configuration in a mechanical device as a container in which the lubricant is accommodated, a physical property parameter of the lubricant, an environmental parameter around the lubricant, and a parameter of time; and

an output step of outputting the evaporation amount calculated in the calculation step.

According to this configuration, it is possible to provide a function for calculating an evaporation amount of a lubricant in a mechanical device.

Although various embodiments have been described above with reference to the drawings, it is needless to mention that the present invention is not limited to these examples. It is apparent for those skilled in the art to which the present disclosure belongs that various changes or modifications are conceivable within the scope recited in the claims, and it is understood that the above falls within the technical scope of the present invention. In addition, the components described in the above embodiments may be combined in any manner without departing from the spirit of the invention.

The present application is based on a Japanese patent application (No. 2023-088047) filed on May 29, 2023, contents of which are incorporated herein by reference.

INDUSTRIAL APPLICABILITY

The present invention has an effect capable of calculating an evaporation amount of a lubricant in a mechanical device such as a rolling bearing, a rotating device, a sliding device, and a motor, in particular, in a device including a path through which the lubricant evaporates and flows to the outside, and may be used for calculation and measurement of the evaporation amount of the lubricant, design of the mechanical device, and the like.

REFERENCE SIGNS LIST

400: rolling bearing
401: outer ring
402: inner ring
403: rolling element
404: cage
405: seal
406: lubricant
800: information processing device

801: processing unit
802: storage unit
803: external IF
804: display unit
805: operation unit
806: communication unit

**Claims**

1. A method for calculating an evaporation amount of a lubricant in a mechanical device to outside, the method comprising:

   a calculation step of calculating the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and
   an output step of outputting the evaporation amount calculated in the calculation step.

2. The calculation method according to claim 1, wherein the calculation formula is defined by the following formula.

   [Math. 1]

$$-\frac{MD}{\rho RT}P_{vap}t = \int_{z_0}^{z_t}\left\{\left(\int_{z}^{z_{out}}\frac{dz}{S(z)}\right)S(z)\right\}dz$$

$$\mathrm{w} = -\rho\int_{z_0}^{z_t}S(z)\,dz$$

   w: evaporation mass [g]
   M: molecular weight [g/mol] of lubricant
   D: diffusion coefficient [$m^2$/s]
   $\rho$: density of lubricant [g/$m^3$]
   R: gas constant [$J\cdot K^{-1}mol^{-1}$]
   T: temperature [K]
   $P_{vap}$: saturated vapor pressure [Pa] at temperature T
   t: time [s]
   $z_t$: liquid level height [m] at time t
   $z_0$: liquid level height [m] at initial time
   z: liquid level height [m] at any time ($z_t < z < z_0$)
   $z_{out}$: height [m] of opening of container
   S(z): cross-sectional area [$m^2$] of container at z

3. The calculation method according to claim 1, wherein the calculation formula is defined by the following formula.

[Math. 2]

$$w = \left( \int_{z_0}^{z_{out}} \frac{dz}{S(z)} \right)^{-1} \cdot \frac{MDP_{vap}}{RT} \cdot t$$

w: evaporation mass [g]
$z_{out}$: height [m] of opening of container
$z_0$: liquid level height [m] at initial time
M: molecular weight [g/mol] of lubricant
D: diffusion coefficient [$m^2$/s]
R: gas constant [$J \cdot K^{-1} mol^{-1}$]
T: temperature [K]
$P_{vap}$: saturated vapor pressure [Pa] at temperature T
t: time [s]
z: liquid level height [m] at any time
S(z): cross-sectional area [$m^2$] of container at z

4. The calculation method according to claim 1, wherein
the evaporation amount of the lubricant is an amount of the lubricant flowing out of the container.

5. The calculation method according to claim 1, wherein
the mechanical device is a rolling bearing, a rotating device, or a sliding device.

6. A method for designing a mechanical device using the calculation method according to any of claims 1 to 5, the method comprising:
a receiving step of receiving designation of the container shape parameter defined by modeling a configuration in the mechanical device as a container in which the lubricant is accommodated.

7. A method for measuring an evaporation amount of a lubricant in a mechanical device using the calculation method according to any of claims 1 to 5, the method comprising:
an acquisition step of acquiring the environmental parameter around the lubricant of the mechanical device, the physical property parameter of the lubricant, and the parameter of time.

8. A device for calculating an evaporation amount of a lubricant in a mechanical device to outside, the device comprising:

a calculation unit configured to calculate the evaporation amount of the lubricant using a calculation formula defined by including a container shape parameter defined by modeling a configuration in the mechanical device as a container in which the lubricant is accommodated, a physical property parameter of the lubricant, an environmental parameter around the lubricant, and a parameter of time; and
an output unit configured to output the evaporation amount calculated by the calculation unit.

9. A program that causes a computer to execute:

a calculation step of calculating an evaporation amount of a lubricant to outside using a calculation formula defined by including a container shape parameter defined by modeling a configuration in a mechanical device as a container in which the lubricant is accommodated, a physical property parameter of the lubricant, an environmental parameter around the lubricant, and a parameter of time; and
an output step of outputting the evaporation amount calculated in the calculation step.

## FIG. 1

## FIG. 2

(a)

(b)

## FIG. 3

(a)                                        (b)

## FIG. 4

(a)                                        (b)

*FIG. 5*

(a)

(b)

*FIG. 6*

(a)

(b)

*FIG. 7*

(a)

(b)

*FIG. 8*

800

801
PROCESSING UNIT

802
STORAGE UNIT

803
EXTERNAL IF

804
DISPLAY UNIT

805
OPERATION UNIT

806
COMMUNICATION UNIT

## INTERNATIONAL SEARCH REPORT

International application No.

**PCT/JP2024/019673**

### A. CLASSIFICATION OF SUBJECT MATTER

*F01M 11/10*(2006.01)i; *F01M 11/12*(2006.01)i; *F16N 29/00*(2006.01)i; *G01N 33/30*(2006.01)i
FI: F01M11/10 Z; G01N33/30; F16N29/00 E; F01M11/12 Z

According to International Patent Classification (IPC) or to both national classification and IPC

### B. FIELDS SEARCHED

Minimum documentation searched (classification system followed by classification symbols)

F01M11/10; F01M11/12; F16N29/00; G01N33/30

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2-278099 A (ISHIKAWAJIMA HARIMA HEAVY IND CO., LTD.) 14 November 1990 (1990-11-14)<br>entire text, all drawings | 1-9 |
| A | JP 2002-146379 A (TONENGENERAL SEKIYU KK) 22 May 2002 (2002-05-22)<br>entire text, all drawings | 1-9 |
| A | WO 2018/212339 A1 (JXTG NIPPON OIL & ENERGY CORPORATION) 22 November 2018 (2018-11-22)<br>entire text, all drawings | 1-9 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **09 August 2024** | **20 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/019673**

| Patent document cited in search report | | | Publication date (day/month/year) | Patent family member(s) | | | Publication date (day/month/year) |
|---|---|---|---|---|---|---|---|
| JP | 2-278099 | A | 14 November 1990 | (Family: none) | | | |
| JP | 2002-146379 | A | 22 May 2002 | (Family: none) | | | |
| WO | 2018/212339 | A1 | 22 November 2018 | US entire text, all drawings | 2020/0080018 | A1 | |
| | | | | EP | 3626805 | A1 | |
| | | | | CN | 110637077 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007107588 A
- JP 2023088047 A